# EUROPEAN PATENT APPLICATION

(11) **EP 4 818 332 A1**
(43) Date of publication of application: **07.10.2026**
(21) Application number: 24896776.2
(22) Date of filing: 29.11.2024
(51) Int. Cl.: C12Q 1/6886, A61K 45/00, A61K 48/00

(54) **USE OF PTPRT AS BIOMARKER AND TARGET FOR PREDICTING EFFICACY OF IMMUNE CHECKPOINT THERAPY IN LUNG CANCER**

(30) Priority: 01.12.2023 CN 202311634054
(71) Applicant: Shanghai Jiao Tong University, Shanghai 200240 (CN); Shanghai Chest Hospital, Shanghai 200030 (CN)
(72) Inventor: XIA, Weiliang, Shanghai 200240 (CN); CHEN, Zhuo, Shanghai 200240 (CN); LI, Ziming, Shanghai 200240 (CN); JI, Wenxiang, Shanghai 200240 (CN)
(74) Representative: Canzler & Bergmeier Patentanwälte Partnerschaft mbB
(86) International application number: PCT/CN2024/135966
(87) International publication number: WO 2025/113697

(57) **Abstract**

The present application relates to the technical field of medicine, and provides a use of PTPRT as a biomarker and a target for predicting the efficacy of immune checkpoint therapy in lung cancer. Further provided is a use of the expression quantity of PTPRT in the prediction or evaluation of the efficacy of immune checkpoint therapy in cancer. Experiments show that the low expression of PTPRT, as a marker for predicting the efficacy of immune checkpoint therapy in lung cancer, can accurately predict progression-free survival (PFS) of lung cancer patients. Inhibition of PTPRT can increase the proportion of tumor-infiltrating CD8+T cells and synergize with immune checkpoint therapy to enhance the anti-tumor effect of an immune checkpoint inhibitor.

## Description

### FIELD OF TECHNOLOGY

The present disclosure relates to the field of medical technology, and particularly relates to the use of PTPRT as a biomarker and a target for predicting the efficacy of immune checkpoint therapy for lung cancer.

### BACKGROUND

Cancer is a leading cause of death worldwide, and the emergence of immune checkpoint therapy has changed the mode of cancer treatment. However, only about 20% of patients continuously benefit from immune checkpoint therapy, and 90% of treated patients suffer adverse reaction events. Therefore, identifying patients who are more likely to benefit from immune checkpoint therapy and enabling precise treatment are of great significance.

At present, the only biomarker approved by the U.S. Food and Drug Administration and most widely used for the efficacy of immune checkpoint therapy is tumor PD-L1 expression. However, PD-L1 expression has limited sensitivity and specificity. Some patients with high PD-L1 expression cannot benefit from immune checkpoint therapy, while about 20% of patients with negative PD-L1 expression respond to immune checkpoint therapy. Tumor mutation burden (TMB), as a biomarker for the efficacy of immune checkpoint therapy, still lacks a unified standard for detection methods and threshold selection. Therefore, there is a need to find a more precise biomarker with clinical translation potential to guide lung cancer patients in receiving immune checkpoint therapy, screen out populations that can benefit from immune checkpoint therapy, and expand the responsive population of immune checkpoint therapy through multi-target combination therapy, thereby bringing survival benefits to more patients.

### SUMMARY

The present disclosure provides a use of PTPRT as a biomarker and a target for predicting the efficacy of immune checkpoint therapy for lung cancer, which can solve problems in the prior art.

It has been found that the accuracy of screening patients by detecting tumor PD-L1 expression needs to be improved. The technical innovation of the present disclosure lies in overcoming the deficiencies of the prior art, and discovering that PTPRT expression can predict the efficacy of immune checkpoint therapy for non-small cell lung cancer and is independent of the method for detecting tumor PD-L1 expression. PTPRT can be detected as a separate indicator to improve the accuracy of patient screening.

A first aspect of the present disclosure provides a use of an expression level of PTPRT in predicting or evaluating the efficacy of immune checkpoint therapy for cancer.

A second aspect of the present disclosure provides a use of a substance for detecting an expression level of PTPRT in preparing a product for evaluating or predicting the efficacy of immune checkpoint therapy for cancer.

A third aspect of the present disclosure provides a kit. The kit includes a substance for detecting an expression level of PTPRT, and the kit has at least one of the following uses: 1) predicting the efficacy of immune checkpoint therapy for lung cancer; and 2) evaluating the efficacy of immune checkpoint therapy for lung cancer.

A fourth aspect of the present disclosure provides a use of a PTPRT inhibitor in preparing a product. The product has at least one of the following effects: 1) increasing infiltration of CD8+ T cells in a lung cancer or colorectal cancer tumor; 2) prolonging the survival period of a lung cancer patient; 3) improving a tumor immune microenvironment of lung cancer or colorectal cancer; and 4) improving the efficacy of an immune checkpoint-related drug in treating lung cancer or colorectal cancer.

A fifth aspect of the present disclosure provides a pharmaceutical composition, including an effective amount of an immune checkpoint-related drug and the PTPRT inhibitor described above.

A sixth aspect of the present disclosure provides a use of the pharmaceutical composition described above in preparing a product for treating lung cancer or colorectal cancer.

The present disclosure provides the following beneficial effects.
1. The present disclosure, for the first time, establishes the relationship between low PTPRT expression and the efficacy of immune checkpoint therapy for lung cancer. Specifically, low PTPRT expression significantly improved progression-free survival (PFS) in lung cancer patients receiving immune checkpoint therapy; low PTPRT expression significantly improved the objective response rate (ORR) in lung cancer patients receiving immune checkpoint therapy; and low PTPRT expression significantly increased infiltration of CD8+ T cells in tumor samples from lung cancer patients. Inhibition of PTPRT improved the tumor immune microenvironment of lung cancer patients. These findings indicate that low PTPRT expression is a novel marker for predicting the efficacy of immune checkpoint therapy in lung cancer patients.
2. The present disclosure found that low PTPRT expression is independent of PD-L1 expression. Low PTPRT expression does not affect PD-L1 expression and can serve as a supplement to PD-L1 expression.
3. By constructing mouse models (preclinical models), the present disclosure found that PTPRT knockout combined with immune checkpoint therapy significantly inhibited tumor growth and reduced tumor size; PTPRT knockout significantly improved the mouse tumor immune microenvironment and increased infiltration of CD8+ T cells; and PTPRT knockout combined with immune checkpoint therapy significantly prolonged the survival of mice.

### BRIEF DESCRIPTION OF THE DRAWINGS

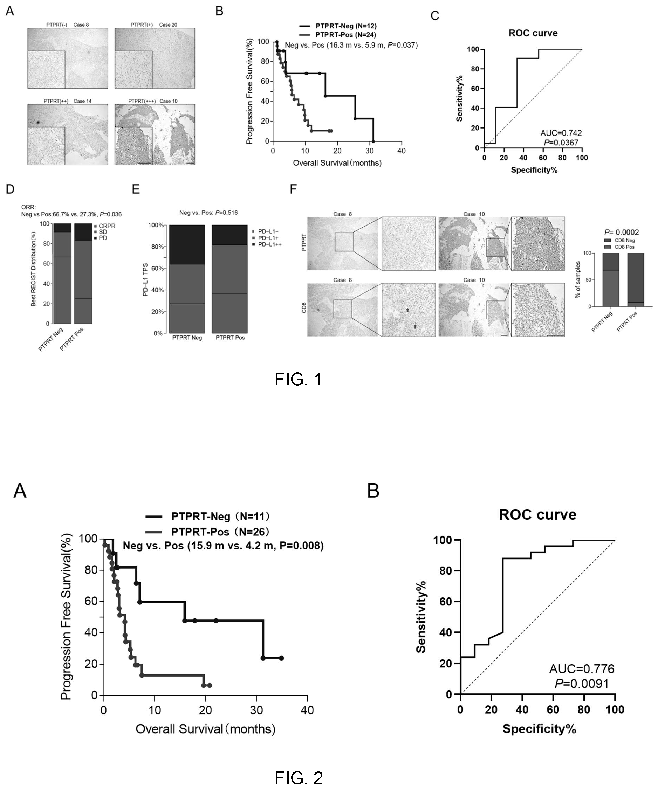

FIG. 1 shows that low PTPRT expression is associated with the efficacy of immune checkpoint therapy for lung cancer. In FIG. 1, panel A shows immunohistochemical staining images of PTPRT (-, +, 2+, 3+) in tumor biopsy samples, and the scale bar is 100 µm; panel B shows analysis of PTPRT expression level and progression-free survival of lung cancer patients receiving immunotherapy; panel C shows an ROC curve of low PTPRT expression for predicting efficacy of immune checkpoint therapy; panel D shows analysis of PTPRT expression level and objective response rate of lung cancer patients receiving immunotherapy; panel E shows analysis of PTPRT expression level and PD-L1 expression level of lung cancer patients receiving immunotherapy; and panel F shows correlation analysis between low and high PTPRT expression groups and CD8+ T cell infiltration.
FIG. 2 shows 37 cases of external validation data. In FIG. 2, panel A shows analysis of PTPRT expression level and progression-free survival in lung cancer patients receiving immunotherapy, and panel B shows an ROC curve of low PTPRT expression for predicting efficacy of immune checkpoint therapy.

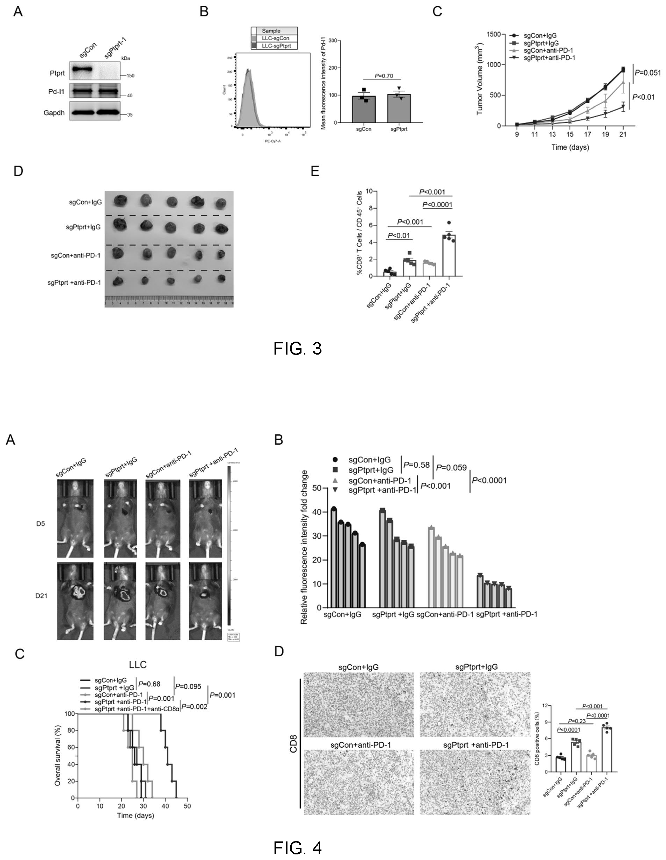

FIG. 3 shows that inhibition of PTPRT expression combination with immunotherapy synergistically enhances the immunotherapeutic effect in a lung cancer animal subcutaneous tumor model. In FIG. 3, panel A shows changes in related proteins after PTPRT knockout in LLC cells; panel B shows changes in fluorescence intensity of PD-L1 in cells after PTPRT knockout in LLC cells detected by flow cytometry; panel C shows changes in tumor volume curves; panel D shows changes in tumor size at the endpoint; and panel E shows a proportion of tumor-infiltrating CD8+ T cells.
FIG. 4 shows that inhibition of PTPRT expression combination with immunotherapy synergistically enhances the immunotherapeutic effect in a lung cancer animal orthotopic tumor model. In FIG. 4, panel A shows results of *in vivo* fluorescence imaging of tumors; panel B shows changes in tumor fluorescence at the endpoint; panel C shows a survival rate of orthotopic tumor-bearing mice; and panel D shows a proportion of tumor-infiltrating CD8+ T cells.

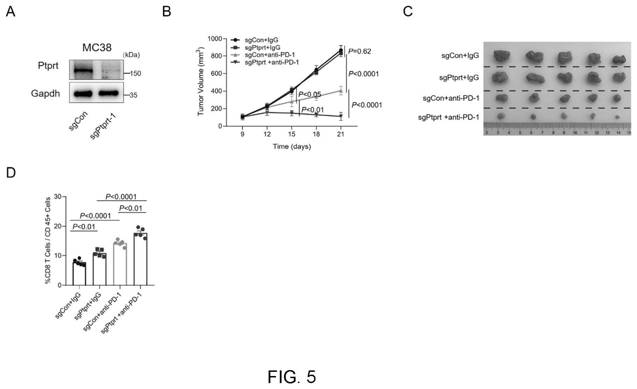

FIG. 5 shows that inhibition of PTPRT expression combination with immunotherapy synergistically enhances the immunotherapeutic effect in a colorectal cancer animal subcutaneous tumor model. In FIG. 5, panel A shows changes in related proteins after PTPRT knockout in MC38 cells; panel B shows changes in tumor volume curves; panel C shows changes in tumor size at the endpoint; and panel D shows a proportion of tumor-infiltrating CD8+ T cells.

### DETAILED DESCRIPTION

To make the objectives, technical solutions, and beneficial effects of the present disclosure clearer, the present disclosure is further described below with reference to examples. It should be understood that the examples are provided only for illustrating the present disclosure and are not intended to limit the scope of the present disclosure. Unless otherwise specified, the test methods used in the following examples were conventional methods and were performed according to techniques or conditions described in technical literature in the art or according to product instructions. A person skilled in the art can readily understand other advantages and effects of the present disclosure from the content disclosed herein. Unless otherwise specified, the materials, reagents, and the like used in the following examples were commercially available.

The present disclosure studied a clinical cohort of patients receiving immune checkpoint therapy from Shanghai Chest Hospital and found that expression of receptor protein tyrosine phosphatase T (PTPRT) was highly correlated with progression-free survival (PFS) of lung cancer patients after receiving immune checkpoint therapy. Low PTPRT expression can be used as a marker for predicting the efficacy of immune checkpoint therapy for lung cancer. PTPRT serves as a target for predicting efficacy of immune checkpoint therapy for lung cancer, and inhibition of PTPRT can effectively improve the immunosuppressive tumor microenvironment by increasing tumor CD8+ T cell infiltration.

A first aspect of the present disclosure provides a use of an expression level of PTPRT in predicting or evaluating the efficacy of immune checkpoint therapy for cancer. The cancer is selected from lung cancer, head and neck squamous cell carcinoma, esophageal cancer, pleural mesothelioma, breast cancer, gastric cancer, hepatocellular carcinoma, colorectal cancer, kidney cancer, urothelial cancer, cervical cancer, endometrial cancer, ovarian cancer, malignant melanoma, malignant lymphoma, and skin cancer. In some embodiments, the cancer is lung cancer or colorectal cancer.

In the use provided by the present disclosure, when the expression level of PTPRT in a sample, as determined by immunohistochemical staining, corresponds to a staining intensity of <1%, good efficacy of immune checkpoint therapy for cancer is predicted; when the expression level of PTPRT in a sample, as determined by immunohistochemical staining, corresponds to a staining intensity of >1%, poor efficacy of immune checkpoint therapy for cancer is predicted.

PTPRT is a receptor protein tyrosine phosphatase T. It is known in the art that PTPRT gene mutations are correlated, to some extent, with the prognosis of lung cancer immunotherapy. The present disclosure discloses, for the first time, the relationship between low expression of the PTPRT protein and the evaluation of the efficacy of immune checkpoint therapy for lung cancer. Specifically, low PTPRT expression significantly increased infiltration of CD8+ T cells in lung cancer tumors; low PTPRT expression significantly improved progression-free survival (PFS) in lung cancer patients; low PTPRT expression significantly improved the objective response rate (ORR) in lung cancer patients; and low PTPRT expression significantly increased infiltration of CD8+ T cells in tumor samples of lung cancer patients. This indicates that low PTPRT expression is a novel marker for predicting the efficacy of immune checkpoint therapy in lung cancer patients.

In the use provided by the present disclosure, the lung cancer includes non-small cell lung cancer. The expression level of PTPRT is the expression level of wild-type PTPRT.

In the use provided by the present disclosure, immune checkpoint therapy for lung cancer includes administering an immune checkpoint-related drug. Further, the immune checkpoint-related drug includes an immune checkpoint inhibitor and/or an immune checkpoint agonist. Further, the immune checkpoint inhibitor includes one or more of a PD-1 inhibitor, a PD-L1 inhibitor, a CTLA-4 inhibitor, a TIM-3 inhibitor, a LAG3 inhibitor, and a TIGIT inhibitor. The immune checkpoint agonist includes one or more of a CD27 agonist, a CD40 agonist, an OX40 agonist, a GITR agonist, a CD137 agonist, a CD28 agonist, and an ICOS agonist. In some embodiments, the immune checkpoint-related drug is a PD-1 inhibitor. Immune checkpoints are expressed on immune cells and can regulate the degree of immune activation, and they play an important role in preventing the occurrence of autoimmunity. Immune checkpoint immunotherapy is a therapeutic method for killing tumor cells by regulating T cell activity through a series of pathways, such as co-inhibitory or co-stimulatory signals.

In the use provided by the present disclosure, the lung cancer tumor microenvironment includes, but is not limited to, a lung cancer tumor immune microenvironment.

In the use provided by the present disclosure, low PTPRT expression is independent of PD-L1 expression. Low PTPRT expression does not affect PD-L1 expression and can serve as a supplement to PD-L1 expression.

In the use provided by the present disclosure, low PTPRT expression means that a staining intensity of <1% in immunohistochemical staining is considered low expression.

A second aspect of the present disclosure provides a use of a substance for detecting an expression level of PTPRT in preparing a product for evaluating or predicting the efficacy of immune checkpoint therapy for cancer.

In the use provided by the present disclosure, the cancer is selected from lung cancer, head and neck squamous cell carcinoma, esophageal cancer, pleural mesothelioma, breast cancer, gastric cancer, hepatocellular carcinoma, colorectal cancer, kidney cancer, urothelial cancer, cervical cancer, endometrial cancer, ovarian cancer, malignant melanoma, malignant lymphoma, and skin cancer. Preferably, the cancer is lung cancer or colorectal cancer.

In the use provided by the present disclosure, the substance for detecting the expression level of PTPRT includes a reagent for detecting a PTPRT protein expression level, detecting PTPRT protein content, or detecting a PTPRT RNA expression level. Specifically, the reagent includes an antibody, a polypeptide, a protein, or a nucleic acid molecule that binds to the PTPRT protein.

The reagent for detecting PTPRT can specifically bind to a specific site on PTPRT, but does not specifically bind to genes other than PTPRT, and the reagent may optionally carry a detectable signal. A method for detecting expression of PTPRT in an analyte by using an antibody specifically binding to PTPRT is well known to a person skilled in the art.

In some embodiments, the substance for detecting the expression level of PTPRT is an antibody, specifically a PTPRT monoclonal antibody.

A third aspect of the present disclosure provides a kit, including a substance for detecting the expression level of PTPRT. The kit has at least one of the following uses: 1) predicting efficacy of immune checkpoint therapy for lung cancer; and 2) evaluating efficacy of immune checkpoint therapy for lung cancer.

The kit includes primers for specifically amplifying a PTPRT gene, a probe for specifically recognizing the PTPRT gene, an antibody or a ligand for specifically binding to RNA encoded by the PTPRT gene, or an antibody or a ligand for specifically binding to a protein encoded by the PTPRT gene. In addition, the kit may further include various reagents required for extracting DNA, extracting RNA, PCR, hybridization, color development, and the like, including but not limited to an extraction solution, an amplification solution, a hybridization solution, an enzyme, a control solution, a color development solution, a washing solution, and the like.

In some embodiments, the kit includes a PTPRT monoclonal antibody. An immunohistochemical staining experiment is performed on a sample, and the efficacy of immune checkpoint therapy for lung cancer is predicted or evaluated by determining the staining intensity of PTPRT. Specifically, when a staining intensity of PTPRT in the sample in immunohistochemical staining is <1%, low expression is determined, and good efficacy of immune checkpoint therapy for lung cancer is predicted; when a staining intensity of PTPRT in the sample in immunohistochemical staining is >1%, high expression is determined, and poor efficacy of immune checkpoint therapy for lung cancer is predicted.

A fourth aspect of the present disclosure provides a use of a PTPRT inhibitor in preparing a product. The product has at least one of the following effects: 1) increasing infiltration of CD8+ T cells in a lung cancer or colorectal cancer tumor; 2) prolonging the survival period of a lung cancer patient; 3) improving the tumor immune microenvironment of lung cancer or colorectal cancer; and 4) improving the efficacy of an immune checkpoint-related drug in treating lung cancer or colorectal cancer.

PTPRT inhibitors include inhibitors, antagonists, blockers, blocking agents, and the like, and these terms may be used interchangeably.

In the use provided by the present disclosure, the PTPRT inhibitor includes a substance that reduces the PTPRT protein expression level or PTPRT protein content, or a substance that inhibits PTPRT gene expression. Further, the substance that inhibits PTPRT gene expression includes a substance that knocks out or silences PTPRT. The substance that knocks out or silences PTPRT includes one or more of a CRISPR gene editing system targeting PTPRT, an interference molecule specifically interfering with the expression of a coding gene of PTPRT, and a homologous recombination substance targeting a loss-of-function mutation of PTPRT.

In some embodiments, the CRISPR gene editing system targeting PTPRT may use a CRISPR/Cas (such as Cas9) system to perform targeted gene editing, so that the PTPRT gene is knocked out in a targeted disease region. Common methods for knocking out the PTPRT gene include co-transfecting sgRNA or a nucleic acid capable of forming sgRNA, Cas9 mRNA, or a nucleic acid capable of forming Cas9 mRNA into a targeted region or targeted cell. After a target site is determined, a known method can be used to introduce the sgRNA and Cas9 into cells. The nucleic acid capable of forming sgRNA is a nucleic acid construct or an expression vector, or the nucleic acid capable of forming the Cas9 mRNA is a nucleic acid construct or an expression vector. These expression vectors are introduced into cells, thereby forming active sgRNA and Cas9 mRNA in the cells. In some embodiments, the CRISPR gene editing system targeting PTPRT includes sgRNA, and an encoding DNA sequence of the sgRNA is shown as SEQ ID NO:1.

In the use provided by the present disclosure, the immune checkpoint-related drug includes an immune checkpoint inhibitor and/or an immune checkpoint agonist. Further, the immune checkpoint inhibitor includes one or more of a PD-1 inhibitor, a PD-L1 inhibitor, a CTLA-4 inhibitor, a TIM-3 inhibitor, a LAG3 inhibitor, and a TIGIT inhibitor; and the immune checkpoint agonist includes one or more of a CD27 agonist, a CD40 agonist, an OX40 agonist, a GITR agonist, a CD137 agonist, a CD28 agonist, and an ICOS agonist. In some embodiments, the immune checkpoint-related drug is a PD-1 inhibitor.

In the use provided by the present disclosure, it was found that PTPRT knockout combined with immune checkpoint therapy significantly inhibited tumor growth and reduced tumor size; PTPRT knockout significantly improved the mouse tumor immune microenvironment and increased infiltration of CD8+ T cells; and PTPRT knockout combined with immune checkpoint therapy significantly prolonged the survival of mice.

A fifth aspect of the present disclosure provides a pharmaceutical composition, including an effective amount of an immune checkpoint-related drug and the PTPRT inhibitor in the foregoing use. The immune checkpoint-related drug includes an immune checkpoint inhibitor and/or an immune checkpoint agonist. Further, the immune checkpoint inhibitor includes one or more of a PD-1 inhibitor, a PD-L1 inhibitor, a CTLA-4 inhibitor, a TIM-3 inhibitor, a LAG3 inhibitor, and a TIGIT inhibitor; and the immune checkpoint agonist includes one or more of a CD27 agonist, a CD40 agonist, an OX40 agonist, a GITR agonist, a CD137 agonist, a CD28 agonist, and an ICOS agonist. In some embodiments, the immune checkpoint-related drug is a PD-1 inhibitor.

The pharmaceutical composition provided by the present disclosure further includes a pharmaceutically acceptable carrier or excipient.

"Pharmaceutically acceptable" means that, when a molecular entity and a composition are appropriately administered to an animal or a human, they do not produce adverse, allergic, or other undesirable reactions.

A "pharmaceutically acceptable carrier or excipient" should be compatible with the immune checkpoint inhibitor and the PTPRT inhibitor, that is, can be mixed with the immune checkpoint inhibitor and the PTPRT inhibitor without substantially reducing the effect of the drug under normal circumstances. Specific examples of substances that can be used as pharmaceutically acceptable carriers or excipients are sugars such as lactose, glucose and sucrose; starches such as corn starch and potato starch; cellulose and derivatives thereof such as sodium methylcellulose, ethyl cellulose and methylcellulose; gum tragacanth powder; malt; gelatin; talc; solid lubricants such as stearic acid and magnesium stearate; calcium sulfate; vegetable oils such as peanut oil, cottonseed oil, sesame oil, olive oil, corn oil and cacao oil; polyhydric alcohols such as propylene glycol, glycerol, sorbitol, mannitol and polyethylene glycol; alginic acid; emulsifiers such as Tween; wetting agents such as sodium lauryl sulfate; colorants; flavoring agents; tableting agents, stabilizers and antioxidants; preservatives; pyrogen-free water; isotonic saline solutions; phosphate buffer solutions and the like. These substances are used as needed to help formulation stability or to help improve activity or bioavailability thereof, or to produce an acceptable taste or odor in the case of oral administration.

The pharmaceutical composition provided by the present disclosure may be adapted to any form of administration, and may be administered orally or parenterally. For example, the pharmaceutical composition may be administered via the lung, via the nose, via the rectum, and/or by intravenous injection, and more specifically may be administered intradermally, subcutaneously, intramuscularly, intra-articularly, intraperitoneally, pulmonarily, buccally, sublingually, nasally, transdermally, vaginally, orally, or parenterally.

A person skilled in the art may select a suitable formulation form according to the route of administration. For example, a formulation form suitable for oral administration may include, but is not limited to, a pill, a tablet, a chewable preparation, a capsule, a granule, a drop, or a syrup. For another example, a formulation form suitable for parenteral administration may include, but is not limited to, a solution, a suspension, a reconstitutable dry preparation, or a spray. For another example, a formulation form suitable for rectal administration may be a suppository.

A sixth aspect of the present disclosure provides a use of the pharmaceutical composition described above in preparing a product for treating lung cancer or colorectal cancer.

A seventh aspect of the present disclosure provides a method for treating lung cancer or colorectal cancer, comprising administering the PTPRT inhibitor or the pharmaceutical composition described above to a subject.

In the method for treating lung cancer provided by the present disclosure, the subject is a mammal. The mammal may be, for example, a rodent, an artiodactyl, a perissodactyl, a lagomorph, a primate, and the like. The primate may be, for example, a monkey, an ape, or Homo sapiens.

The present disclosure is further described below through examples, but the scope of the present disclosure is not limited thereby.

### Example 1

### Application of PTPRT in predicting efficacy of immune checkpoint therapy for lung cancer

Case selection in this example: this example was a retrospective clinical study. The study was conducted in accordance with the principles of the Declaration of Helsinki, and informed consent was obtained from all subjects. This example mainly included patients with stage IIIC/IV lung cancer who received immune checkpoint therapy at Shanghai Chest Hospital from January 2020 to June 2022, and the follow-up date ended on December 31, 2022. The treatment received was anti-PD-1 therapy or anti-PD-1 therapy plus chemotherapy.

Efficacy evaluation during treatment of the patients was performed according to Response Evaluation Criteria in Solid Tumors (RECIST), version 1.1. Efficacy evaluation indicators included complete response (CR), partial response (PR), stable disease (SD), and progressive disease (PD). Progression-free survival (PFS) was defined as the time from the start of immune checkpoint therapy to disease progression or death. The objective response rate was a proportion of patients in whom tumor volume shrinkage reached 30% and maintained a minimum time-limit requirement, and was a sum of proportions of CR (complete disappearance of a tumor) and PR (tumor shrinkage reaching 30% or more), excluding SD.

Immunohistochemical staining was performed on samples to detect the expression percentage of PTPRT. IHC staining intensity was divided into: -, <1%; +, 1%-10%; ++, 10%-50%; +++, >50%.

Specific operations were as follows.
a. Slide baking: paraffin sections were placed on a slide-baking machine at 60°C for 30 min.
b. Section dewaxing and rehydration: three jars of xylene were prepared (three washes). The baked paraffin sections were quickly placed into glass jars and soaked in each jar for 5 min. Then the sections were passed through absolute ethanol twice, 2 min each time, and then sequentially passed through 95%, 90%, 80% and 70% ethanol, staying in each concentration for 5 min. Finally, the sections were soaked in PBS buffer three times, 3 min each time.
c. Section fixation: the dewaxed and rehydrated sections were soaked in 4% PFA solution for 10 min, and then soaked in PBS buffer three times, 3 min each time.
d. Membrane permeabilization: the fixed sections were soaked in 0.3% Triton-100 for 10 min, and then soaked in PBS buffer three times, 3 min each time.
e. Removal of oxidoreductase: the permeabilized sections were soaked in 3% hydrogen peroxide solution for 10 min to remove endogenous oxidoreductase in the tissue. The sections were then soaked in PBS buffer three times, 3 min each time.
f. Blocking: after the oxidoreductase was removed from the sections, the liquid around the tissue on the slides was wiped dry, and a circle was drawn around the tissue with an immunohistochemistry pen to prevent liquid dripped on the section tissue in subsequent steps from flowing out. The sections were placed in a light-protected wet box, and a blocking solution containing 10% goat serum was then dripped onto the section tissue. After standing at room temperature for 1 h, the blocking solution on the section tissue was aspirated, and the sections were then soaked in PBS buffer three times, 3 min each time.
g. Primary antibody incubation: the antibody was diluted with antibody diluent according to the instructions of the primary antibody (R&D, product No. AF3697), and then the primary antibody was dripped onto the surface of the blocked tissue and incubated in a refrigerator at 4°C overnight.
h. Secondary antibody incubation: the primary antibody liquid on the surface of the sections was aspirated, and the sections were then soaked in PBS buffer three times, 3 min each time. The prepared secondary antibody corresponding to the species of the primary antibody (Jackson ImmunoResearch, product No. 705-005-003) was dripped onto the section tissue. After incubation at room temperature for 1 h, the sections were soaked in PBS buffer three times, 3 min each time.
i. DAB color development: DAB color development solution was dripped onto the section tissue. A preliminary experiment may first be performed to confirm a suitable reaction time under a microscope before a formal experiment is carried out. Generally, the staining time was 1-5 min, and finally, the sections were gently rinsed with slow-running tap water to terminate color development.
j. Nuclear staining: the sections after DAB color development were placed in hematoxylin solution for counterstaining, so that nuclei of all cells were stained (blue). Generally, staining at room temperature for 2-5 min was sufficient, and finally, the sections were gently rinsed with slow-running tap water to terminate color development.
k. Mounting: after the above steps were completed, the sections were mounted for long-term preservation. The slides were sequentially placed in 70%, 80%, 90%, 95% and 100% ethanol for gradient dehydration for 2 min, dehydrated again in 100% ethanol, and then soaked in xylene twice, 5 min each time. A small amount of neutral resin was dripped onto the section tissue, and a coverslip was placed thereon while avoiding bubbles. Finally, the slides were fully dried in a fume hood and placed in a slide box for subsequent observation and photographing.

### 1. Survival curve

The results are shown in panel A of FIG. 1. According to PTPRT expression of the patients, the patients were divided into PTPRT low-expression (-) patients and PTPRT high-expression (+, ++, +++) patients. The results are shown in panels B and D of FIG. 1. A survival curve and an objective response rate were plotted according to progression-free survival time of the patients. The analysis results indicated that, at the same follow-up time, the progression-free survival of patients with low PTPRT expression was significantly prolonged (16.3 months vs. 5.9 months, P=0.037), and the objective response rate was also significantly increased (66.7% vs. 27.3%, P=0.036). The ROC curve in panel C of FIG. 1 showed that low PTPRT expression predicted efficacy of immune checkpoint therapy with high specificity and sensitivity (AUC=0.742, P=0.0367).

Another 37 cases of other data were used for external validation. Case selection in this example: this example was a retrospective clinical study. The study was conducted in accordance with the principles of the Declaration of Helsinki, and informed consent was obtained from all subjects. This example mainly included patients with stage IIIC/IV lung cancer who received immune checkpoint therapy at Shanghai Chest Hospital from March 2019 to June 2022, and the follow-up date ended on March 31, 2023. The treatment received was PD-1 inhibitor therapy or PD-1 inhibitor therapy plus chemotherapy.

Efficacy evaluation during treatment of the patients was performed according to Response Evaluation Criteria in Solid Tumors (RECIST), version 1.1. Progression-free survival (PFS) was defined as the time from the start of immune checkpoint therapy to disease progression or death. Immunohistochemical staining was performed on samples to detect the expression percentage of PTPRT. IHC staining intensity was divided into: -, <1%; +, 1%-100%.

The results are shown in FIG. 2. At the same follow-up time, the progression-free survival of patients with low PTPRT expression was significantly increased compared with patients with high PTPRT expression (15.9 months vs. 4.2 months, P=0.008). A suitable ROC curve showed that low PTPRT expression had high specificity and sensitivity.

The ROC curve in panel B of FIG. 2 showed that low PTPRT expression predicted efficacy of immune checkpoint therapy with high specificity and sensitivity (AUC=0.776, P=0.0091).

### 2. Relationship between PTPRT expression and tumor immune microenvironment

The tumor PD-L1 proportion and CD8+ T cell infiltration were evaluated according to the results of immunohistochemical staining. PD-L1 was evaluated using the common tumor proportion score (TPS). A sample with CD8+ T cell infiltration was defined as a positive (+) sample, and a sample without CD8+ T cell infiltration was defined as a negative (-) sample. The results are shown in panels D and E of FIG. 1. Low PTPRT expression showed no statistically significant difference from PD-L1 expression level (P=0.516), while the proportion of CD8+ T cell infiltration in patients with low PTPRT expression was increased (P=0.0002).

In summary, the efficacy of immune checkpoint therapy for a lung cancer patient to be tested can be predicted or assisted in being predicted by detecting the expression level of PTPRT in the tumor tissue of the lung cancer patient to be tested, and the determination criteria are as follows.

The efficacy of immune checkpoint therapy in patients in the PTPRT low-expression group was better than, or potentially better than, that in patients in the PTPRT high-expression group. Therefore, PTPRT can be used as a biomarker for evaluating or auxiliarily evaluating the efficacy of immune checkpoint therapy.

### Example 2

A PTPRT inhibitor combined with immune checkpoint therapy can synergistically enhance the anti-lung cancer effect of immunotherapy

The PTPRT inhibitor may be a substance that inhibits PTPRT gene expression, silences or knocks out the PTPRT gene, or may be a substance that inhibits or reduces PTPRT protein content and/or activity.

### 1. Selection of cell line and animal

The mouse LLC cell line used in this study was obtained from the ATCC cell resource center in the United States.

Six-week-old C57BL/6 female mice were used in this study. All mice used in the experiment were housed in an SPF-level environment. All operations in the study strictly complied with the rules and regulations formulated by the Animal Ethics Committee.

### 2. Construction of LLC cells with PTPRT knockout

According to the PTPRT gene sequence and the CRISPR-Cas9 principle, the PTPRT gene was knocked out in LLC cells. The encoding DNA sequence of the sgRNA designed for knocking out the PTPRT gene was 5'-CAGCAACTGCGGGTATAGCG-3' (SEQ ID NO:1), and the encoding DNA sequence of the control sgRNA was 5'-GAACAGTCGCGTTTGCGACT-3' (SEQ ID NO:2). After LLC cells were transduced with lentiviruses for PTPRT knockout and blank control, LLC cells with PTPRT gene knockout and control LLC cells were obtained. Changes in cellular PTPRT and PD-L1 were detected by Western blotting, and changes in PD-L1 on the cell membrane surface were detected by flow cytometry.

### 3. Mouse lung cancer subcutaneous transplanted tumor model

LLC cells with PTPRT gene knockout and control LLC cells (one million cells) were subcutaneously injected into the right dorsal side of wild-type C57BL/6 female mice (6 weeks old). Tumor volume was calculated using the following formula: (width × width × length)/2. PD-1 inhibitor treatment (BE0273, BioXcell) and IgG isotype control (BE0089, BioXcell) were intraperitoneally administered at 150 µg each time. Administration was started on day 9 and performed once every 3 days, and tumors were collected on day 21 for flow cytometry analysis. When tumors were collected, mice were sacrificed by carbon dioxide anesthesia and dissected for sampling to alleviate animal suffering.

The results were as follows. The protein expression level of PTPRT in LLC cells with PTPRT knockout was significantly decreased, while the protein level of PD-L1 was not significantly changed (panel A of FIG. 3). Flow cytometry detection found that fluorescence intensity of PD-L1 on the cell membrane surface of LLC cells with PTPRT knockout was not significantly changed (panel B of FIG. 3). In the mouse lung cancer subcutaneous transplanted tumor model, after treatment with PTPRT knockout combined with a PD-1 inhibitor, tumor growth rate and size were significantly decreased compared with the control treatment group (PD-1 inhibitor group) and the knockout group (PTPRT knockout group) (panels C and D of FIG. 3), and the proportion of CD8+ T cell infiltration was increased (panel E of FIG. 3).

The above results indicated that PTPRT knockout or inhibition of PTPRT expression did not affect tumor cell PD-L1 expression. PTPRT knockout or inhibition of PTPRT expression in synergy with a PD-1 inhibitor could significantly inhibit tumor growth, improve the efficacy of immune checkpoint therapy, increase infiltration of CD8+ T cells in tumors, and enhance the anti-tumor effect of immunotherapy.

### Example 3

A PTPRT inhibitor combined with immune checkpoint therapy can synergistically enhance the anti-lung cancer effect of immunotherapy

### 1. Selection of cell line and animal

The mouse LLC-luc cell line used in this study was obtained from the ATCC cell resource center in the United States.

Six-week-old C57BL/6 female mice were used in this study. All mice used in the experiment were housed in an SPF-level environment. All operations in the study strictly complied with the rules and regulations formulated by the Animal Ethics Committee.

### 2. Construction of LLC-luc cells with PTPRT knockout

According to the PTPRT gene sequence and the CRISPR-Cas9 principle, the PTPRT gene was knocked out in LLC cells. The encoding DNA sequence of the sgRNA designed for knocking out the PTPRT gene was 5'-CAGCAACTGCGGGTATAGCG-3' (SEQ ID NO:1), and the encoding DNA sequence of the control sgRNA was 5'-GAACAGTCGCGTTTGCGACT-3' (SEQ ID NO:2). After lentiviruses for PTPRT knockout and blank control were transfected into LLC cells, LLC cells with PTPRT gene knockout and control LLC cells were obtained.

### 3. Mouse lung cancer orthotopic transplanted tumor model

Mice were anesthetized with isopentane using an animal gas anesthesia machine. A 3-mm incision was made on the dorsal side of the left lung of a mouse, 0.5 cm below the scapula. After the subcutaneous tissue and muscle were separated, movement of the lung could be observed. A cell suspension (Matrigel: PBS=1: 4) of the stably transfected cells obtained in step 2, that is, LLC cells with PTPRT gene knockout and control LLC cells (eight hundred thousand cells), in a total volume of 50 µl, was directly injected into the left lung using an insulin syringe (BD), and the incision was sutured. *In vivo* fluorescence imaging was performed on day 5 to observe modeling. PD-1 inhibitor treatment (BE0273, BioXcell) and IgG isotype control (BE0089, BioXcell) were intraperitoneally administered at 150 µg each time. Administration was started on day 9 and performed once every 3 days, and *in vivo* fluorescence imaging was performed at the experimental endpoint on day 21. For a survival experiment, PD-1 inhibitor treatment (BE0273, BioXcell) and IgG isotype control (BE0089, BioXcell) were intraperitoneally administered at 150 µg each time from day 7 after modeling until the mice died. An anti-CD8α antibody (200 µg, BE0061, BioXCell) was intraperitoneally injected twice a week from 7 days before modeling.

The results were as follows. In the mouse lung cancer orthotopic transplanted tumor model, after treatment with PTPRT knockout combined with a PD-1 inhibitor, tumor growth rate and size were significantly decreased compared with the control treatment group (PD-1 inhibitor group) and the knockout group (PTPRT knockout group) (panels A and B of FIG. 4), and the proportion of CD8+ T cell infiltration was increased (panel C of FIG. 4). Meanwhile, PTPRT knockout combined with a PD-1 inhibitor significantly prolonged the overall survival of mice (panel D of FIG. 4).

The above results indicated that PTPRT knockout or inhibition of PTPRT expression in combination with a PD-1 inhibitor could significantly inhibit tumor growth, prolong survival, increase infiltration of CD8+ T cells in tumors, and enhance the anti-tumor effect of immunotherapy.

### Example 4

A PTPRT inhibitor combined with immune checkpoint therapy can synergistically enhance the anti-colorectal cancer effect of immunotherapy

The PTPRT inhibitor may be a substance that inhibits PTPRT gene expression, silences or knocks out the PTPRT gene, or may be a substance that inhibits or reduces PTPRT protein content and/or activity.

### 1. Selection of cell line and animal

The mouse MC38 cell line used in this study was obtained from the ATCC cell resource center in the United States.

Six-week-old C57BL/6 female mice were used in this study. All mice used in the experiment were housed in an SPF-level environment. All operations in the study strictly complied with the rules and regulations formulated by the Animal Ethics Committee.

### 2. Construction of MC38 cells with PTPRT knockout

According to the PTPRT gene sequence and the CRISPR-Cas9 principle, the PTPRT gene was knocked out in MC38 cells. The encoding DNA sequence of the sgRNA designed for knocking out the PTPRT gene was 5'-CAGCAACTGCGGGTATAGCG-3' (SEQ ID NO:1), and the encoding DNA sequence of the control sgRNA was 5'-GAACAGTCGCGTTTGCGACT-3' (SEQ ID NO:2). After MC38 cells were transfected with lentiviruses for PTPRT knockout and blank control , MC38 cells with PTPRT gene knockout and control MC38 cells were obtained.

### 3. Mouse colorectal cancer subcutaneous transplanted tumor model

MC38 cells with PTPRT gene knockout and control MC38 cells (one million cells) were subcutaneously injected into the right dorsal side of wild-type C57BL/6 female mice (6 weeks old). Tumor volume was calculated using the following formula: (width × width × length)/2. PD-1 inhibitor treatment (BE0273, BioXcell) and IgG isotype control (BE0089, BioXcell) were intraperitoneally administered at 150 µg each time. Administration was started on day 9 and performed once every 3 days, and tumors were collected on day 21 for flow cytometry analysis. When tumors were collected, mice were sacrificed by carbon dioxide anesthesia and dissected for sampling to alleviate animal suffering.

The results were as follows. The protein expression level of PTPRT in MC38 cells with PTPRT knockout was significantly decreased (panel A of FIG. 5). In the mouse colorectal cancer subcutaneous transplanted tumor model, after treatment with PTPRT knockout combined with a PD-1 inhibitor, tumor growth rate and size were significantly decreased compared with the control treatment group (PD-1 inhibitor group) and the knockout group (PTPRT knockout group) (panels B and C of FIG. 5), and the proportion of CD8+ T cell infiltration was increased (panel D of FIG. 5).

PTPRT knockout or inhibition of PTPRT expression in combination with a PD-1 inhibitor could significantly inhibit tumor growth, improve efficacy of immune checkpoint therapy, increase infiltration of CD8+ T cells in tumors, and enhance the anti-tumor effect of immunotherapy.

In summary, the predictive biomarker provided by the present disclosure can maximize the efficacy of immune checkpoint therapy, identify patient populations that are likely to benefit from immune checkpoint therapy, and PTPRT inhibition can expand the population responsive to immune checkpoint therapy, thereby bringing survival benefits to more patients.

The above examples are merely illustrative of the principle and effects of the present disclosure and are not intended to limit the present disclosure. Any person skilled in the art may modify or change the above examples without departing from the scope of the present disclosure. Therefore, all equivalent modifications or changes completed by a person of ordinary skill in the art without departing from the technical ideas disclosed by the present disclosure should still be covered by the claims of the present disclosure.

## Claims

1. Use of an expression level of PTPRT in predicting or evaluating efficacy of immune checkpoint therapy for cancer.

2. The use according to claim 1, wherein the cancer is selected from lung cancer, head and neck squamous cell carcinoma, esophageal cancer, pleural mesothelioma, breast cancer, gastric cancer, hepatocellular carcinoma, colorectal cancer, kidney cancer, urothelial cancer, cervical cancer, endometrial cancer, ovarian cancer, malignant melanoma, malignant lymphoma, and skin cancer; preferably, the cancer is lung cancer or colorectal cancer.

3. The use according to claim 1, wherein, when a staining intensity of PTPRT in a sample in immunohistochemical staining is <1%, low expression is determined, and good efficacy of immune checkpoint therapy for cancer is predicted; and when a staining intensity of PTPRT in the sample in immunohistochemical staining is >1%, high expression is determined, and poor efficacy of immune checkpoint therapy for cancer is predicted.

4. The use according to claim 1, wherein the expression level of PTPRT is an expression level of wild-type PTPRT; and/or immune checkpoint therapy for lung cancer comprises administering an immune checkpoint-related drug; the immune checkpoint-related drug comprises an immune checkpoint inhibitor and/or an immune checkpoint agonist; preferably, the immune checkpoint inhibitor comprises one or more of a PD-1 inhibitor, a PD-L1 inhibitor, a CTLA-4 inhibitor, a TIM-3 inhibitor, a LAG3 inhibitor and a TIGIT inhibitor; the immune checkpoint agonist comprises one or more of a CD27 agonist, a CD40 agonist, an OX40 agonist, a GITR agonist, a CD137 agonist, a CD28 agonist and an ICOS agonist; more preferably, the immune checkpoint-related drug is a PD-1 inhibitor.

5. Use of a substance for detecting an expression level of PTPRT in preparing a product for evaluating or predicting efficacy of immune checkpoint therapy for cancer.

6. The use according to claim 5, wherein the cancer is selected from lung cancer, head and neck squamous cell carcinoma, esophageal cancer, pleural mesothelioma, breast cancer, gastric cancer, hepatocellular carcinoma, colorectal cancer, kidney cancer, urothelial cancer, cervical cancer, endometrial cancer, ovarian cancer, malignant melanoma, malignant lymphoma, and skin cancer; preferably, the cancer is lung cancer or colorectal cancer;
and/or the substance for detecting the expression level of PTPRT comprises a reagent for detecting a PTPRT protein expression level, detecting PTPRT protein content, or detecting a PTPRT RNA expression level.

7. The use according to claim 6, wherein the reagent comprises an antibody, a polypeptide, a protein, or a nucleic acid molecule that binds to PTPRT protein; preferably, the antibody is a PTPRT monoclonal antibody.

8. A kit, wherein the kit comprises a substance for detecting an expression level of PTPRT, and the kit has at least one of the following uses:
1) predicting efficacy of immune checkpoint therapy for lung cancer; and
2) evaluating efficacy of immune checkpoint therapy for lung cancer.

9. Use of a PTPRT inhibitor in preparing a product, wherein the product has at least one of the following effects:
1) increasing infiltration of CD8+ T cells in a lung cancer or colorectal cancer tumor;
2) prolonging a survival period of a lung cancer patient;
3) improving a tumor immune microenvironment of lung cancer or colorectal cancer; and
4) improving efficacy of an immune checkpoint-related drug in treating lung cancer or colorectal cancer.

10. The use according to claim 9, wherein the PTPRT inhibitor comprises a substance that reduces a PTPRT protein expression level or PTPRT protein content, or a substance that inhibits PTPRT gene expression; wherein the substance that inhibits PTPRT gene expression comprises a substance that knocks out or silences PTPRT; wherein the substance that knocks out or silences PTPRT comprises one or more of: a CRISPR gene editing system targeting PTPRT, an interference molecule specifically interfering with expression of a coding gene of PTPRT, and a homologous recombination substance targeting a loss-of-function mutation of PTPRT;
and/or the immune checkpoint-related drug comprises an immune checkpoint inhibitor and/or an immune checkpoint agonist; preferably, the immune checkpoint inhibitor comprises one or more of a PD-1 inhibitor, a PD-L1 inhibitor, a CTLA-4 inhibitor, a TIM-3 inhibitor, a LAG3 inhibitor, and a TIGIT inhibitor; the immune checkpoint agonist comprises one or more of a CD27 agonist, a CD40 agonist, an OX40 agonist, a GITR agonist, a CD137 agonist, a CD28 agonist, and an ICOS agonist; more preferably, the immune checkpoint-related drug is a PD-1 inhibitor.

11. The use according to claim 10, wherein the CRISPR gene editing system targeting PTPRT comprises sgRNA, and an encoding DNA sequence of the sgRNA is shown as SEQ ID NO:1.

12. A pharmaceutical composition, comprising an effective amount of an immune checkpoint-related drug and the PTPRT inhibitor in the use according to any one of claims 9 to 11.

13. The pharmaceutical composition according to claim 12, wherein the immune checkpoint-related drug comprises an immune checkpoint inhibitor and/or an immune checkpoint agonist; preferably, the immune checkpoint inhibitor comprises one or more of a PD-1 inhibitor, a PD-L1 inhibitor, a CTLA-4 inhibitor, a TIM-3 inhibitor, a LAG3 inhibitor and a TIGIT inhibitor; the immune checkpoint agonist comprises one or more of a CD27 agonist, a CD40 agonist, an OX40 agonist, a GITR agonist, a CD137 agonist, a CD28 agonist and an ICOS agonist; more preferably, the immune checkpoint-related drug is a PD-1 inhibitor.

14. Use of the pharmaceutical composition according to any one of claims 12 to 13 in preparing a product for treating lung cancer or colorectal cancer.
